(19) Europäisches Patentamt
European Patent Office
Office européen des brevets

(11) Numéro de publication : **0 138 875**
**B1**

(12) **FASCICULE DE BREVET EUROPÉEN**

(45) Date de publication du fascicule du brevet : **27.05.87**

(51) Int. Cl.⁴ : **C 08 F216/12**, A 61 L 15/00 // (C08F216/12, 220:04)

(21) Numéro de dépôt : **84901089.7**

(22) Date de dépôt : **16.03.84**

(86) Numéro de dépôt international : **PCT/FR 84/00066**

(87) Numéro de publication internationale : **WO/8403706 (27.09.84 Gazette 84/23)**

(54) **COPOLYMERE ALLYLOLIGOSACCHARIDE-ACRYLIQUE SALIFIE, PROCEDE DE PREPARATION DE COPOLYMERE ET APPLICATION COMME SUPER-ABSORBANT.**

(30) Priorité : **18.03.83 FR 8304488**

(43) Date de publication de la demande : **02.05.85 Bulletin 85/18**

(45) Mention de la délivrance du brevet : **27.05.87 Bulletin 87/22**

(84) Etats contractants désignés : **BE DE GB LU NL**

(56) Documents cités :
**FR-A- 1 088 642**
**FR-A- 2 305 452**
**FR-A- 2 334 692**
**US-A- 2 606 881**

(73) Titulaire : **BEGHIN-SAY SOCIETE ANONYME**
**F-59239 Thumeries (FR)**

(72) Inventeur : **TOURNIER, Hervé**
**Le Riondet**
**F-74520 Valleiry (FR)**

(74) Mandataire : **Quéré, Jean Pierre**
**BEGHIN-SAY Service Propriété Industrielle 54, Avenue Hoche**
**F-75008 Paris (FR)**

**EP 0 138 875 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

L'invention concerne le domaine des produits absorbant les liquides notamment le sang et l'urine.

Elle a pour objet un copolymère allyloligosaccharide-acrylique salifié et son utilisation comme superabsorbant dans l'hygiène corporelle, l'agriculture et comme stabilisateur d'humidité.

Elle a également pour objet un procédé de préparation dudit copolymère allyloligosaccharide-acrylique.

Etat de la technique

Deux documents connus du déposant décrivent déjà des copolymères allyloligosaccharide-acrylique.

Le brevet US 2 606 881 décrit la polymérisation d'allyldisaccharide comme l'allylsaccharose avec un dérivé-acrylique.

L'allyldisaccharide est préparé par ethérification de tout ou partie des huit groupements alcool par un groupement allylique.

Ainsi huit fonctions allyliques au plus peuvent être portées par un disaccharide, soit un degré de substitution (DS) de huit au maximum. Le brevet US 2 606 881 décrit un allylsaccharose contenant 6,5 groupes allyliques par molécule. Les produits obtenus après copolymérisation du dérivé allyldisaccharide avec le dérivé acrylique, dans un rapport en poids voisin de 1, ne présentent toutefois aucune propriété absorbante significative quand ceux-ci sont plongés, selon un test normalisé, décrit par Goldstein et Pierre. (Marketing Technologize Service — September 16-18, 1981 — Absorbent Products conference) dans de l'eau salée à 35 °C, pendant 30 minutes.

Le brevet US 2 798 053 décrit l'utilisation de copolymères allylsaccharose-acrylique comme résine échangeuse d'ions. Les allylsaccharoses ont un degré de substitution moyen (nombre de groupements allyle par molécule de saccharose) supérieur à 5 et ne sont utilisés qu'en très faible rapport molaire. En outre, ils ne présentent aucune propriété absorbante selon le test précédent quand ils sont copolymérisés avec un dérivé acrylique. Il est même écrit (exemple 3) que quand on utilise une plus grande quantité d'allylsaccharose on obtient des polymères très durs gonflant peu. L'invention va à l'encontre de l'enseignement de ce document. En effet, l'objet de l'invention est au contraire de proposer une nouvelle famille de copolymère allyloligosaccharide-acrylique présentant d'excellentes propriétés absorbantes.

Description de l'invention

Selon l'invention le copolymère allyloligosaccharide-acrylique salifié est caractérisé en ce qu'il est constitué sous forme combinée chimiquement d'au moins un allyloligosaccharide de degré de substitution moyen inférieur à 4 par molécule et d'au moins un dérivé acrylique présentant des fonctions carboxyle est partiellement ou totalement salifiées, ledit copolymère ayant un degré de rétention après 30 min dans l'eau salée à 10 g/l supérieur à 8 g d'eau salée par gramme de copolymère.

Pour un oligosaccharide on appelle degré de substitution moyen ($\overline{DS}$) le nombre de groupements allyles ayant remplacé des groupements hydroxyles dans cette molécule. Il est d'ailleurs bien évident que lors de la synthèse des allyloligosaccharides on obtient un mélange et que le degré de substitution moyen est calculé à partir de ce mélange.

On appelle dans cette demande de brevet oligosaccharide les sucres présentant un à dix motifs glucidiques liés chimiquement conformément à la nomenclature du Chemical abstract. Les motifs glucidiques peuvent correspondre à des chaînes contenant cinq ou six atomes de carbone qui sont de préférence cycliques comme le glucose ou le fructose et leurs isomères stéréochimiques.

Ces oligosaccharides proviennent par exemple de l'hydrolyse de substances polymériques comme l'amidon, le glycogène, la cellulose.

Parmi les oligosaccharides convenant pour l'invention les mono, di, tri et tétra saccharides seront préférés et parmi ceux-ci les disaccharides seront préférés.

Parmi ceux-ci on peut citer à titre indicatif le saccharose ou sucrose en anglais, le maltose, le cellobiose, le lactose. Le saccharose est préféré pour la présente invention.

La préparation des allyloligosaccharides s'effectue de manière connue, par exemple par éthérification, au moyen d'un halogénure d'allyle d'une fonction alcool. Le schéma réactionnel est le suivant :

$$R - OH + X - CH_2 - \underset{\underset{R_1}{|}}{C} = CH_2 \xrightarrow{NaOH} R - O - CH_2 - \underset{\underset{R_1}{|}}{C} = CH_2$$

R = radical oligosaccharide
$R_1$ = H, alkyle de $C_1$ à $C_4$
X = Cl, Br

Ce procédé est illustré pour l'allylation du saccharose par le brevet US 2 719 970. Il est clair dans ce schéma, que le dérivé allylique fixé sur l'oligosaccharide peut être substitué sur le carbone en β. Néanmoins, il est préférable dans le cadre de l'invention d'utiliser le dérivé allylique où $R_1$ = H c'est-à-dire le dérivé allylique non substitué.

Les documents cités ci-dessus ne décrivent que des allyloligosaccharides de degré de substitution moyen élevé (supérieur à 5,0) qui ne peuvent conduire aux copolymères de l'invention.

On entend par dérivé acrylique tous les dérivés ayant une double liaison liée à un groupe carboxyle ou un groupe susceptible de conduire à un groupe carboxyle par réaction d'hydrolyse (ester, nitrile, amide), ladite double liaison n'étant pas substituée sur le carbone β, comme indiqué selon la formule ci-dessous :

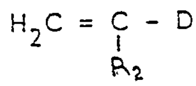

D = COOH, $COOR_3$, $CONH_2$, CN,
$R_2$ étant l'atome d'hydrogène ou un groupement alkyle de $C_1$ à $C_4$,
$R_3$ étant un groupement alkyle de $C_1$ à $C_4$.

Parmi ceux-ci on peut citer l'acide acrylique ou méthacrylique, l'acrylate, le méthacrylate d'alkyle saturé de $C_1$ à $C_8$ linéaire ou ramifié. On préférera, toutefois, l'acide acrylique ou méthacrylique. On peut également citer les diacrylates résultant de l'estérification d'un diol court par l'acide acrylique mais ces derniers conduisent à des produits moins performants.

Il est également possible d'utiliser des dérivés acrylamide et de préférence l'acrylamide ou le méthacrylamide seul ou en mélange, ainsi que l'acrylonitrile.

Ces dérivés acryliques, dans le copolymère, sont, au moins partiellement, sous forme salifiée, de préférence par un cation alcalin comme le potassium, sodium, ou encore l'ammonium. Le sodium et le potassium sont préférés dans le cadre de l'invention. De préférence 10 % au moins des groupements carboxyle seront salifiés. Une des propriétés physiques qui permettent de caractériser le copolymère de l'invention réside dans ses propriétés absorbantes. On a donc défini un test d'absorption dont les conditions sont les suivantes :

Le copolymère, placé dans une enveloppe neutre, est immergé à 35 °C pendant 30 minutes dans une solution de NaCl à 10 g/l puis le produit est centrifugé à 1 200 g pendant une minute. On mesure les poids avant et après immersion et le degré de rétention se calcule par comparaison avec un essai témoin. Ce degré de rétention $R_{30}$ doit être supérieur à 8.

De préférence, le degré de substitution moyen par motif monomérique sera inférieur à 3,0 et supérieur ou égal à 1,0.

Afin d'obtenir des copolymères allyloligosaccharide-acrylique présentant de meilleures propriétés d'absorption il est préférable que ceux-ci soient constitués d'un mélange d'allyloligosaccharides de degré de substitution moyen supérieur ou égal à 5 et d'allyloligosaccharides de degré de substitution moyen par motif réticulé inférieur ou égal à 3, la moyenne étant bien entendu inférieure à 4. Cette remarque s'applique pour les copolymères préférés ($1,5 \leq \overline{DS} \leq 3$), c'est-à-dire qu'il est préférable que le mélange soit constitué d'allyloligosaccharides de $\overline{DS} \leq 1,5$ et $\overline{DS} \geq 3$.

Il a en effet été trouvé de manière surprenante que cette répartition améliorait le comportement des copolymères quant à leur capacité d'absorption.

Quant aux proportions respectives d'allyloligosaccharides et de dérivés acrylique qui constituent le copolymère il est préférable qu'elles correspondent à un rapport molaire allylique/acrylique compris entre 0,005 et 1, soit en poids dans le cas du couple allylsaccharose de $\overline{DS}$ = 2/acide acrylique, un rapport de 0,03 à 6.

Dans le cas des allyldisaccharide-monoacryliques qui sont les copolymères préférés de l'invention on peut définir les proportions limites préférées en poids à savoir :

| | |
|---|---|
| allyl disaccharide | 5 à 30 % |
| dérivé mono acrylique | 70 à 95 % |
| et de préférence allyldisaccharide | 8 à 25 % |
| dérivé monoacrylique | 75 à 92 % |

Dans le cas des allyldisaccharides de degré de substitution faible (voisin de 1) il sera préférable d'avoir une proportion supérieure d'allyldisaccharide en mole à savoir :

| | |
|---|---|
| allyldisaccharide | 15 à 30 % |
| dérivé monoacrylique | 85 à 70 % |

L'invention a également pour objet un procédé de préparation d'un copolymère allyloligosaccharide

salifié comme décrit ci-dessus caractérisé en ce que l'on fait réagir un dérivé acrylique et un mélange aqueux d'allyloligosaccharide de degré de substitution moyen inférieur à 4 à une température supérieure à 10 °C, en présence d'un initiateur de polymérisation radicalaire en présence d'eau et que le copolymère obtenu est salifié partiellement ou totalement par une base minérale forte, puis séché.

Il est préférable afin d'obtenir de meilleurs rendements de polymérisation que l'eau du mélange aqueux soit présente dans une proportion de 0,5 g par gramme d'allylsaccharose bien qu'il soit tout à fait possible d'effectuer la réaction en présence d'une quantité extrêmement minime (0,01 g/g) d'eau par gramme d'allylsaccharose.

Il est évident que tout ce qui a été écrit précédemment à propos des allyloligosaccharides et des dérivés acrylique s'applique également à cette partie de l'invention.

La température est également un paramètre critique et bien que la réaction s'effectue à 10 °C il est nettement préférable d'effectuer la réaction à une température comprise entre 40 et 90 °C. Les rendements de polymérisation sont plus élevés ainsi que la vitesse de réaction. Le catalyseur est également en quantité plus faible. Par ailleurs, les propriétés superabsorbantes des produits augmentent.

De préférence, le mélange aqueux de polymérisation est à un pH légèrement acide.

Il est essentiel que la salification des fonctions COOH soit effectuée après la polymérisation et non pas avant. En effet, dans ce dernier cas les copolymères obtenus présentent des qualités médiocres. On utilisera avantageusement la soude ou la potasse.

Il a été trouvé qu'il était tout à fait possible d'effectuer la réaction en présence d'oligosaccharide non allylé. Selon une variante de l'invention on emploiera donc comme produit de départ un mélange d'allyldisaccharide ayant la composition suivante en poids :

| | |
|---|---|
| disaccharide | 10 à 20 % |
| mono-allyldisaccharide | 25 à 35 % |
| di-allyldisaccharide | 20 à 30 % |
| tri-allyldisaccharide | 10 à 20 % |
| tetra-allyldisaccharide | 5 à 7 % |
| allyldisaccharide de degré de substitution supérieur ou égal à 5 | < 3,5 % |

Ce mélange convient parfaitement pour le but de l'invention qui est d'obtenir des produits absorbants.

Il a été trouvé, en outre, qu'il était tout à fait possible d'utiliser comme produit de départ le mélange réactionnel brut qui provient de l'allylation du disaccharide.

Ainsi une économie importante peut être faite à ce niveau du procédé. Le mélange brut a de préférence la composition suivante en poids :

30 à 50 % d'eau
20 à 30 % d'halogénure d'un métal alcalin
20 à 50 % d'allyldisaccharide.

De préférence, l'allyldisaccharide est l'allylsaccharose.

Après réaction de copolymérisation radicalaire du mélange d'allylsaccharose et du dérivé acrylique, le copolymère est précipité sous agitation par du méthanol, filtré, lavé au méthanol et séché en étuve à une température voisine de 50 °C.

Il est, en outre, très intéressant de faire subir au copolymère séché une étape supplémentaire de purification en le traitant par exemple avec de l'eau ; le produit ainsi gélifié est ensuite traité au méthanol, filtré puis séché à l'étuve. Ce traitement conduit à des propriétés d'absorption supérieures à celles du copolymère de départ.

Il est, en outre, possible de lyophiliser le copolymère purifié sous forme gélifiée. Ce traitement supplémentaire conduit à des produits ayant des qualités d'absorption du sang exceptionnelles jamais atteintes à la connaissance du déposant.

Les copolymères de l'invention trouvent une application particulièrement intéressante comme additifs superabsorbants dans les produits pour l'hygiène corporelle comme les serviettes périodiques, les couches pour bébés ou pour adultes incontinents.

En outre, ces copolymères peuvent être appliqués à l'agriculture comme produits retenant l'humidité, particulièrement utiles dans les zones chaudes du globe.

Ils peuvent également être appliqués comme stabilisateur d'humidité dans des enceintes à degré hygrométrique contrôlé.

La préparation des allyldisaccharides s'effectue par exemple par étherification d'une fonction alcool au moyen d'un halogénure d'allyle. Le schéma réactionnel est le suivant :

$$R - OH + X - CH_2 - \underset{R_1}{\underset{|}{C}} = CH_2 \xrightarrow{NaOH} R - O - CH_2 - \underset{R_1}{\underset{|}{C}} = CH_2$$

R = radical disaccharide
$R_1$ = H, alkyle de $C_1$ à $C_4$
X = Cl, Br

On utilisera de préférence le dérivé allylique non substitué c'est-à-dire $R_1$ = H.

Ce procédé est illustré pour l'allylation du saccharose notamment par le brevet US 2 719 970 et JACS 6 746.

L'invention est maintenant illustrée par les exemples suivants :

Synthèse de l'allylsaccharose

Exemple 1

Dans un réacteur de 1 litre, on introduit 278,1 g de saccharose de P.M. 342 soit 0,81 mole, 66 g de NaOH dans 75 ml d'eau ; on coule en 1 heure 199,2 g de bromure d'allyle soit 1,65 mole sous agitation en en maintenant la température à 85 °C, on maintient l'agitation et la température pendant 2 heures et jusqu'à l'obtention d'un pH voisin de 7, on ajoute alors 200 ml d'eau.

La composition du mélange réactionnel brut est la suivante en poids :

| | |
|---|---|
| sucre non réagi | 8,5 % |
| Allylsaccharoses | 35,0 % |
| Na Br et produits secondaires | 21,5 % |
| Eau | 35 % |

avec une répartition molaire, mesurée par CLHP, suivante des allylsaccharoses :

| | |
|---|---|
| sucre non réagi | 18 % |
| allylsaccharose DS 1 | 30,9 % |
| allylsaccharose DS 2 | 26,5 % |
| allylsaccharose DS 3 | 15,1 % |
| allylsaccharose DS 4 | 6,5 % |
| allylsaccharose DS 5 | 2,2 % |
| allylsaccharose DS 6 et au-delà | 0,8 % |

Le degré de substitution moyen du mélange est donc de 2,08.

Il est possible d'effectuer une élimination des produits secondaires alcool allylique et dérivé allylique par entraînement réalisé par distillation d'une partie de l'eau du mélange réactionnel. Toutefois cette purification n'est pas nécessaire pour l'utilisation à laquelle ces produits sont destinés et le procédé s'en trouve sensiblement simplifié.

Exemple 2

Afin de minimiser la formation d'allylsaccharose de DS élevé, cet essai a été effectué avec un rapport molaire bromure d'allyle/saccharose de 1, c'est-à-dire deux fois plus faible que pour l'essai décrit à l'exemple 1.

Quantités engagées :

| | | |
|---|---|---|
| saccharose | 278,1 g | (0,81 mole) |
| bromure d'allyle | 96,6 g | (0,8 mole) |
| NaOH | 50 g | (1,25 mole) |

dans 60 ml d'eau

La coulée du bromure d'allyle est effectuée en deux heures à une température de 80 à 90 °C et après refroidissement la masse réactionnelle est amenée à pH7 par une addition de 160 ml d'acide chlorhydrique dilué.

La masse totale obtenue est de 620 g.

La composition en poids du mélange réactionnel est la suivante :

| | |
|---|---|
| allylsaccharoses + sucre non réagi | 48,5 % |
| NaBr | 13 % |
| NaCl | 4 % |
| Eau | 34,5 % |

avec une répartition molaire mesurée suivante des allylsaccharoses :

| | |
|---|---|
| sucre non réagi | 40,6 % |
| allylsaccharose DS 1 | 36,5 % |
| allylsaccharose DS 2 | 16,5 % |
| allylsaccharose DS 3 | 5 % |
| allylsaccharose DS 4 | 1,1 % |
| allylsaccharose DS 5 | 0,2 % |
| allylsaccharose DS 6 | 0,03 % |

On peut ainsi disposer d'un mélange qui, s'il contient plus de saccharose non réagi, présente une quantité relative plus importante de DS 1 par rapport aux allylsaccharoses de DS plus élevé.

## Exemple 3

### Etape de purification de l'allylsacchrose

Obtention de la coupe DS 1,2,3

Le mélange réactionnel brut (820 g) obtenu selon l'exemple 1 est placé dans un extracteur liquide-liquide et extrait en continu par 1 litre d'éther diéthylique (le chloroforme peut être également utilisé). Après 24 heures d'extraction, la phase éthérée est séparée de la phase aqueuse et séchée en présence de sulfate de sodium.

L'évaporation de l'éther permet d'obtenir une fraction d'allylsaccharoses de masse égale à 57 g et présentant un DS moyen de 3,9 (ensemble des allylsaccharoses de DS > 3), répartis de la façon suivante :

| | |
|---|---|
| allylsaccharose DS 3 | 16 g |
| allylsaccharose DS 4 | 27 g |
| allylsaccharose DS 5 | 10 g |
| allylsaccharose DS 6 | < 3 g |
| allylsaccharose DS 7 | < 1 g |
| allylsaccharose DS 8 | < 0,2 g |

L'eau de la phase aqueuse est évaporée sous pression réduite et on ajoute de l'éthanol absolu de façon à précipiter le bromure de sodium, produit secondaire de la réaction de condensation. Celui-ci est séparé par filtration et l'opération est répétée trois fois successivement de façon à éliminer ainsi la majeure partie du NaBr. On récupère ainsi un mélange de masse : 278 g, constitué de sucre non réagi, et d'allylsaccharoses de DS < 3 (DS moyen = 1,40) et répartis de la façon suivante :

| | |
|---|---|
| sucre non réagi | 50 g |
| allylsaccharose DS 1 | 96 g |
| allylsaccharose DS 2 | 91 g |
| allylsaccharose DS 3 | 41 g |

Une chromatographie liquide préparative effectuée sur ce mélange a permis d'isoler sous forme pure (95 %) l'allylsaccharose de DS 1.

## Exemple 4

### Synthèse du copolymère allylsaccharose-acrylique

Cet essai peut être considéré comme la meilleure réalisation.

On place dans un ballon de 1 l, à 80° sous agitation, 20 g d'acide acrylique (0,277 mole) avec 30 g du mélange brut d'allylsaccharose de l'exemple 1, (comportant donc 10 g environ d'allylsaccharose soit 0,025 mole) et 80 g d'eau supplémentaire. On ajoute alors le système catalytique constitué successivement de 4 ml des solutions aqueuses de $Fe\ SO_4$ à 1 %, $Na\ HSO_3$ à 5 %, $(NH_4)_2\ S_2\ O_8$ à 10 %. On observe une gélification exothermique très rapide.

Le copolymère obtenu sous forme de gel élastique est neutralisé par une solution de potasse méthanolique en quantité stoechiométrique, 18,3 g (0,278 mole) dans 100 ml de méthanol (KOH à 85 %) sous forte agitation mécanique.

Puis le copolymère salifié précité est traité par du méthanol au mixer, filtré 3 fois successivement, séché à l'étuve à une température de 40° à 50 °C.

On obtient 36,5 g de copolymère sec correspondant à un rendement pondéral de 90 % rapporté au produit actif mis en jeu.

Purification

10 g du copolymère précédent sont gélifiés dans 100 g d'eau, précipités par du méthanol, filtré et séché à l'étuve à 40°-50 °C. Le rendement de purification est de 84 %. Le produit ainsi purifié présente des performances accrues :

Exemple 5

Variation de la quantité d'eau lors de la polymérisation

Trois essais 5a, 5b, 5c ont été réalisés dans les mêmes conditions que l'essai de l'exemple 4 en faisant varier la quantité d'eau supplémentaire présente dans le milieu à savoir :
(Pour mémoire, l'exemple 4 engageait 80 gr d'eau supplémentaire.)

5a : 0 g
5b : 40 g
5c : 160 g

Les rendements sont les suivants :

5a : 46 %
5b : 75 %
5c : 69 %

Les rendements de purification :

5b : 81 %
5c : 77 %

Exemple 6

Variation de la température

Deux essais ont été réalisés dans les mêmes conditions que l'essai de l'exemple 4 en faisant seulement varier la température de copolymérisation à savoir :

6a : 39 °C
6b : 50 °C

Le rendement est de :

6a : 18 %
6b : 43 %

Exemple 7

Proportions de fonctions COOH salifiées

Le procédé est le même que celui de l'exemple 4 mis à part le fait que l'on a fait varier le degré de salification du polymère en faisant varier la quantité de base minérale introduite. La soude a été utilisée pour ces essais à savoir :

7a : (0,278 mole × 0,75)
7b : (0,278 mole × 0,50)
7c : (0,278 mole × 0,33)

Les rendements sont bien entendu identiques si l'on tient compte de la modification de poids entraînée par une salification plus ou moins complète.

Exemple 8

Synthèse avec un allylsaccharose de DS réel = 1 (selon l'exemple 3).

On fait réagir 10 g d'acide acrylique soit 0,14 mole avec 5 g d'allylsaccharose pur de degré de substitution exact 1 (ce produit a été obtenu par chromatographie préparative) soit 0,013 mole en

solution éthanolique à 50 % en présence de 4 ml de solution catalytique comme dans les exemples précédants et 40 g d'eau. On observe une gélification moins rapide et un gel est moins « ferme » que lorsqu'on travaille avec le mélange total d'allylsaccharose. On maintient une température de 80 °C pendant 1 h 45. La neutralisation est assurée par 9,2 g de potasse dans 50 ml de méthanol. Le produit est précipité, lavé, séché à 40 °C. Le rendement est de 75 %.

Exemple 9

Synthèse avec un allylsaccharose de DS moyen réel > 3.

L'essai est effectué dans les mêmes conditions que l'exemple 8 avec un allylsaccharose de degré de substitution moyen de 3,9 obtenu par extraction à l'éther du mélange brut. Le rendement est de 66 %.

Exemple 10

Essai avec l'acrylamide

50 g d'acrylamide et 50 g d'allylsaccharoses (mélange réactionnel brut de l'exemple 1) sont dissous dans 400 ml d'eau et chauffés sous agitation à 70-80 °C. On ajoute alors successivement :

10 ml d'$H_2SO_4$ 10 %
10 ml d'isopropanol
10 ml d'une solution aqueuse de persulfate d'ammonium à 10 %.

Le mélange gélifie, puis prend en masse 4 à 5 minutes après l'addition du persulfate d'ammonium.

On ajoute alors une quantité stœchiométrique de soude sous forme de solution aqueuse 1 et on porte à reflux le mélange réactionnel pendant 3 heures pour hydrolyser les fonctions $—CO—NH_2$ en —COONa. Le mélange réactionnel est alors traité après refroidissement dans du méthanol et le copolymère qui se sépare est filtré, puis séché à température ambiante sous pression réduite. Le rendement est de 78 %.

Propriétés absorbantes des copolymères obtenus par les essais décrits dans les exemples précédents.

On mesure la rétention d'eau salée à une concentration de 10 g/l après immersion à 20 °C d'une enveloppe inerte constituée par 0,50 g de produits répartis sur 36 $cm^2$ puis centrifugation à 1 200 g pendant 1 minute. La rétention du produit est déterminée par différence des rétentions entre une serviette avec et une autre sans produit. Selon que les serviettes auront séjourné 3 ou 30 minutes dans le bain d'eau salée, les tests seront appelés respectivement $R_3$ et $R_{30}$. Ces tests ont été effectués sur les produits non purifiés et sur les produits purifiés, pour la plupart.

Deux essais ont également été effectués avec deux produits commerciaux :

* SANWET (amidon polyacrylate), fabriqué par la Société japonaise SANYO.
* AQUA KEEP (polyacrylate), fabriqué par la Société japonaise SEITETSU-PUK JAPON.

Les résultats sont rassemblés dans le tableau ci-dessous.

Tableau

| | EX | $R_3$ | $R_{30}$ | $R_{30}$ après purification |
|---|---|---|---|---|
| Référence | 4 | 25 | 35 | 45 |
| Variation quantité eau | 5a | 11,5 | 20 | - |
| | 5b | 18 | 20 | 23 |
| | 5c | 13,5 | 20 | 32 |
| Variation température | 6a | 7,5 | 10 | - |
| | 6b | 23 | 27 | - |
| Variation taux salification | 7a | 16 | 25 | |
| | 7b | 26 | 31 | |
| | 7c | 13 | 23 | |
| DS = 1 | 8 | 2,5 | 2,5 | - |
| DS = 3,9 | 9 | 9 | 9 | - |

Tableau (Suite)

| | EX | $R_3$ | $R_{30}$ | $R_{30}$ après purification |
|---|---|---|---|---|
| Allylsacchrose acrylamide | 10 | | 17,5 | 37,5 |
| SANWET (R) | | | 32 | |
| AQUA KEEP (R) | | | 33 | |

Application des copolymères décrits dans les essais précédents comme additifs à rétention amélioré dans les tampons absorbants.

On dispose sur une serviette périodique de surface 36 $cm^2$ 1 g de copolymère obtenu selon l'exemple 4 sur une surface de 36 $m^2$. La serviette est ensuite plongée dans un mélange simulant le sang menstruel à 20 °C ayant la composition suivante en masse :

8.5 % de sang de bœuf
15 % d'eau salée à 9 g NaCl/l
25 mg/l d'héparinate

soit pendant 3 mm ($R_3$)
soit pendant 30 mm ($R_{30}$)

Après centrifugation, on pèse et on fait le rapport avec les résultats obtenus avec une serviette périodique ne comportant pas de copolymères.

Un essai a également été fait avec le même copolymère lyophilisé.

Les résultats sont les suivants :

| | |
|---|---|
| $R_3$ | 4.5 |
| $R_{30}$ | 10.5 |
| $R_{30}$ Produit lyophilisé | 20 |
| $R_{30}$ SANWET (R) | 13 |
| $R_{30}$ AQUA KEEP (R) | 11 |
| $R_{30}$ lyophilisé SANWET (R) | 12 |
| $R_{30}$ lyophilisé AQUA KEEP (R) | 12 |

## Revendications

1. Copolymère allyloligosaccharide-acrylique salifié caractérisé en ce qu'il est constitué sous forme combinée chimiquement d'au moins un allyloligosaccharide de degré de substitution moyen inférieur à 4 par molécule d'oligosaccharide et d'au moins un dérivé acrylique présentant des fonctions carboxyle partiellement ou totalement salifiées, ledit copolymère ayant un degré de rétention après 30 min dans l'eau salée à 10 g/l supérieur à 8 g d'eau salée par gramme de copolymère.

2. Copolymère allyloligosaccharide-acrylique salifié selon la revendication 1, caractérisé en ce que le degré de substitution moyen par molécule d'oligosaccharide est inférieur à 3 et supérieur ou égal à 1.

3. Copolymère allyloligosaccharide-acrylique salifié selon l'une des revendications 1 ou 2 caractérisé en ce que l'allyloligosaccharide est un allyldisaccharide.

4. Copolymère allyloligosaccharide-acrylique salifié selon la revendication 3, caractérisé en ce que l'allyldisaccharide est l'allylsaccharose.

5. Copolymère allyloligosaccharide-acrylique salifié selon la revendication 1, caractérisé en ce que le dérivé acrylique est choisi parmi l'acide acrylique ou méthacrylique, l'amide de l'acide acrylique ou méthacrylique, seuls ou en mélange.

6. Copolymère allyloligosaccharide-acrylique salifié selon la revendication 1, caractérisé en ce que l'allyloligosaccharide de degré de substitution moyen inférieur à 4 est constitué d'un allyloligosaccharide de degré de substitution moyen inférieur à 3, et d'un allyloligosaccharide de degré de substitution moyen supérieur à 5.

7. Copolymère allyloligosaccharide-acrylique salifié selon l'une des revendications précédentes caractérisé en ce qu'au moins 10 % des groupements carboxyles sont salifiés.

8. Copolymère allyloligosaccharide-acrylique salifié selon l'une des revendications précédentes caractérisé en ce que le rapport molaire allylique : acrylique est compris entre 0.005 et 1.

9. Copolymère allyloligosaccharide-acrylique salifié selon l'une des revendications 3 à 8, caractérisé en ce que le copolymère est constitué en poids de :

| | |
|---|---|
| allyldisaccharide | 5 à 30 % |
| dérivé monoacrylique | 70 à 95 % |

10. Copolymère allyloligosaccharide-acrylique salifié selon la revendication 9, caractérisé en ce que le copolymère est constitué en poids de :

| | |
|---|---|
| allyldisaccharide | 8 à 25 % |
| dérivé monoacrylique | 75 à 92 % |

11. Procédé de préparation d'un copolymère allyloligosaccharide-acrylique salifié selon l'une des revendications précédentes, caractérisé en ce que l'on fait réagir un dérivé acrylique et un mélange d'allyloligosaccharides de degré de substitution moyen inférieur à 4 par molécule d'oligosaccharide à une température supérieure à 10 °C, en présence d'un initiateur de polymérisation radicalaire et que le copolymère obtenu est salifié partiellement ou totalement par une base minérale forte puis séché.

12. Procédé de préparation d'un copolymère allyloligosaccharide-acrylique salifié selon la revendication 11, caractérisé en ce que le mélange d'allyloligosaccharides est aqueux.

13. Procédé de préparation d'un copolymère allyloligosaccharide-acrylique salifié selon la revendication 12, caractérisé en ce que le mélange aqueux d'allyloligosaccharide comporte au moins 5 g d'eau pour 10 g d'allyloligosaccharide.

14. Procédé de préparation d'un copolymère allyloligosaccharide-acrylique salifié selon la revendication 11, caractérisé en ce que la réaction de la copolymérisation est effectuée à une température supérieure ou égale à 40 °C et inférieure à 90 °C.

15. Procédé de préparation d'un copolymère allyloligosaccharide-acrylique salifié selon la revendication 11, caractérisé en ce que le mélange d'allyloligosaccharide est un mélange d'allyldisaccharide ayant la composition suivante en masse :

| | |
|---|---|
| saccharose | 10 à 20 % |
| mono-allyldisaccharide | 25 à 35 % |
| di-allyldisaccharide | 20 à 30 % |
| tri-allyldisaccharide | 10 à 20 % |
| tetra-allyldisaccharide | 5 à 7 % |
| allyldisaccharide de degré de substitution supérieur ou égal à 5 | $< 3,5$ % |

16. Procédé de préparation d'un copolymère allyloligosaccharide-acrylique salifié selon la revendication 15, caractérisé en ce que le mélange aqueux d'allyldisaccharide contient en masse :

30 à 50 % d'eau
20 à 30 % d'halogénure d'un métal alcalin
20 à 50 % d'allyldisaccharide

17. Procédé de préparation d'un copolymère allyloligosaccharide-acrylique salifié selon l'une des revendications 12 à 16 caractérisé en ce que le mélange aqueux est à un pH légèrement inférieur à 7.

18. Procédé de préparation d'un copolymère allyloligosaccharide-acrylique salifié selon la revendication 11, caractérisé en ce que le séchage est assuré par lavage au méthanol puis passage en étuve.

19. Procédé de préparation d'un copolymère allyloligosaccharide-acrylique salifié selon la revendication 11, caractérisé en ce que le copolymère est ensuite gélifié, précipité et purifié au méthanol ou à l'éthanol.

20. Procédé de préparation d'un copolymère allyloligosaccharide-acrylique salifié selon la revendication 11 ou 19, caractérisé en ce que le copolymère est, en outre, lyophylisé.

21. Utilisation des copolymères allyloligosaccharide-acryliques salifiés selon l'une des revendications 1 à 10 comme additifs dans les tampons absorbants des serviettes hygiéniques, couches pour bébés, couches pour adultes incontinents.

## Claims

1. Allyloligosaccharide-acrylic copolymer, in salt form, characterised in that it comprises a chemically combined form of at least one allyloligosaccharide of a mean degree of substitution of less than 4 per molecule of oligosaccharide and at least one acrylic derivative containing carboxyl groups which are partially or completely in salt form, the said copolymer having a degree of retention of more than 8 g of salt water per gram of copolymer after 30 min in water containing 10 g of salt/l.

2. Allyloligosaccharide-acrylic copolymer, in salt form, according to Claim 1, characterised in that the mean degree of substitution per molecule of oligosaccharide is less than 3 and more than or equal to 1.

3. Allyloligosaccharide-acrylic copolymer, in salt form, according to either of Claims 1 or 2,

characterised in that the allyloligosaccharide is an allyldisaccharide.

4. Allyloligosaccharide-acrylic copolymer, in salt form, according to Claim 3, characterised in that the allyldisaccharide is allylsucrose.

5. Allyloligosaccharide-acrylic copolymer, in salt form, according to Claim 1, characterised in that the acrylic derivative is chosen from acrylic or methacrylic acid, acrylamide or methacrylamide, by themselves or mixed.

6. Allyloligosaccharide-acrylic copolymer, in salt form, according to Claim 1, characterised in that the allyloligosaccharide of a mean degree of substitution of less than 4 comprises an allyloligosaccharide of a mean degree of substitution of less than 3 and of an allyloligosaccharide of a mean degree of substitution of more than 5.

7. Allyloligosaccharide-acrylic copolymer, in salt form, according to one of the preceding claims, characterised in that at least 10 % of the carboxyl groups are in salt form.

8. Allyloligosaccharide-acrylic copolymer, in salt form, according to one of the preceding claims, characterised in that the allylic/acrylic molar ratio is between 0.005 and 1.

9. Allyloligosaccharide-acrylic copolymer, in salt form, according to one of Claims 3 to 8, characterised in that the copolymer comprises by weight :

| | |
|---|---|
| allyldisaccharide | 5 to 30 % |
| monoacrylic derivative | 70 to 95 % |

10. Allyloligosaccharide-acrylic copolymer, in salt form, according to Claim 9, characterised in that the copolymer comprises by weight :

| | |
|---|---|
| allyldisaccharide | 8 to 25 % |
| monoacrylic derivative | 75 to 92 % |

11. Process for the preparation of an allyloligosaccharide-acrylic copolymer, in salt form, according to one of the preceding claims, characterised in that an acrylic derivative and a mixture of allyloligosaccharides of a mean degree of substitution of less than 4 per molecule of oligosaccharide are reacted at a temperature above 10 °C in the presence of a radical polymerization initiator and that the copolymer obtained is converted partially or completely into salt form by means of a strong inorganic base and is then dried.

12. Process for the preparation of an allyloligosaccharide-acrylic copolymer, in salt form, according to Claim 11, characterised in that the mixture of allyloligosaccharides is aqueous.

13. Process for the preparation of an allyloligosaccharide-acrylic copolymer, in salt form, according to Claim 12, characterised in that the aqueous allyloligosaccharide mixture contains at least 5 g of water per 10 g of allyloligosaccharide.

14. Process for the preparation of an allyloligosaccharide-acrylic copolymer, in salt form, according to Claim 11, characterised in that the copolymerisation reaction is performed at a temperature above or equal to 40 °C and below 90 °C.

15. Process for the preparation of an allyloligosaccharide-acrylic copolymer, in salt form, according to Claim 11, characterised in that the allyloligosaccharide mixture is an allyldisaccharide mixture having the following composition on a mass basis :

| | |
|---|---|
| sucrose | 10 to 20 % |
| monoallyldisaccharide | 25 to 35 % |
| diallyldisaccharide | 20 to 30 % |
| triallyldisaccharide | 10 to 20 % |
| tetraallyldisaccharide | 5 to 7 % |
| allyldisaccharide of a degree of substitution of more than or equal to 5 | $<$ 3.5 % |

16. Process for the preparation of an allyloligosaccharide-acrylic copolymer, in salt form, according to Claim 15, characterised in that the aqueous allyldisaccharide mixture contains, on a mass basis :

30 to 50 % of water
20 to 30 % of an alkali metal halide
20 to 50 % of allyldisaccharide

17. Process for the preparation of an allyloligosaccharide-acrylic copolymer, in salt form, according to one of Claims 12 to 16, characterised in that the aqueous mixture is at a pH slightly below 7.

18. Process for the preparation of an allyloligosaccharide-acrylic copolymer, in salt form, according to Claim 11, characterised in that the drying is effected by washing with methanol followed by a period in an oven.

19. Process for the preparation of an allyloligosaccharide-acrylic copolymer, in salt form, according

to Claim 11, characterised in that the copolymer is then gelled, precipitated and purified with methanol or ethanol.

20. Process for the preparation of an allyloligosaccharide-acrylic copolymer, in salt form, according to Claim 11 or 19, characterised in that the copolymer is, additionally, freeze-dried.

21. Use of the allyloligosaccharide-acrylic copolymers in salt form according to one of Claims 1 to 10 as additives in absorbent pads in sanitary towels, babies' nappies or napkins for incontinent adults.

**Patentansprüche**

1. Allyloligosaccharid-Acryl-Copolymer in Salzform, dadurch gekennzeichnet, daß es, chemisch gebunden, aus wenigstens einem Allyloligosaccharid eines mittleren Substitutionsgrades von unter 4 pro Oligosaccharid-Molekül und wenigstens einem Acrylderivat, dessen Carboxylgruppen teilweise oder vollständig in die Salzform übergeführt sind, besteht, wobei das Copolymer nach 30 Minuten in Salzwasser eines Salzgehaltes von 10 g/l einen Retentionsgrad von über 8 g Salzwasser pro Gramm Copolymer aufweist.

2. Copolymer nach Anspruch 1, dadurch gekennzeichnet, daß der mittlere Substitutionsgrad pro Oligosaccharid-Molekül unter 3 und über 1 liegt oder gleich 1 ist.

3. Copolymer nach Anspruch 1 oder 2, dadurch gekennzeichnet, daß das Allyloligosaccharid ein Allyldisaccharid ist.

4. Copolymer nach Anspruch 3, dadurch gekennzeichnet, daß das Allyldisaccharid eine Allylsaccharose ist.

5. Copolymer nach Anspruch 1, dadurch gekennzeichnet, daß das Acrylderivat ausgewählt ist aus : Acrylsäure, Methacrylsäure, Acrylsäureamid, Methacrylsäureamid und Gemischen davon.

6. Copolymer nach Anspruch 1, dadurch gekennzeichnet, daß das Allyloligosaccharid eines mittleren Substitutionsgrades von unter 4 aus einem Allyloligosaccharid eines mittleren Substitutionsgrades unter 3 und einem Allyloligosaccharid eines mittleren Substitutionsgrades von über 5 besteht.

7. Copolymer nach einem der vorhergehenden Ansprüche, dadurch gekennzeichnet, daß wenigstens 10 % der Carboxylgruppen in die Salzform übergeführt sind.

8. Copolymer nach einem der vorhergehenden Ansprüche, dadurch gekennzeichnet, daß das Molverhältnis von Allyl zu Acryl zwischen 0,005 und 1 liegt.

9. Copolymer nach einem der Ansprüche 3 bis 8, dadurch gekennzeichnet, daß es aus

5 bis 30 Gew.-% Allyldisaccharid und
70 bis 95 Gew.-% eines Monoacrylderivats

besteht.

10. Copolymer nach Anspruch 9, dadurch gekennzeichnet, daß es aus

8 bis 25 Gew.-% Allyldisaccharid und
75 bis 92 Gew.-% eines Monoacrylderivats

besteht.

11. Verfahren zur Herstellung eines Allyloligosaccharid-Acryl-Copolymers in Salzform nach einem der vorhergehenden Ansprüche, dadurch gekennzeichnet, daß man ein Acrylderivat und ein Gemisch von Allyloligosacchariden mit einem mittleren Substitutionsgrad von unter 4 pro Molekül Oligosaccharid in Gegenwart eines Radikale bildenden Polymerisationsinitiators bei einer Temperatur von über 10 °C reagieren läßt, das erhaltene Copolymer teilweise oder ganz mit einer starken mineralischen Base in die Salzform überführt und dann trocknet.

12. Verfahren zur Herstellung eines Copolymers nach Anspruch 11, dadurch gekennzeichnet, daß das Allyloligosaccharid-Gemisch wäßrig ist.

13. Verfahren zur Herstellung eines Copolymers nach Anspruch 12, dadurch gekennzeichnet, daß das wäßrige Allyloligosaccharid-Gemisch wenigstens 5 g Wasser pro 10 g Allyloligosaccharid enthält.

14. Verfahren zur Herstellung eines Copolymers nach Anspruch 11, dadurch gekennzeichnet, daß die Copolymerisationsreaktion bei einer Temperatur von über oder gleich 40 °C und unter 90 °C durchgeführt wird.

15. Verfahren zur Herstellung eines Copolymers nach Anspruch 11, dadurch gekennzeichnet, daß das Allyloligosaccharid-Gemisch ein Gemisch folgender Zusammensetzung, bezogen auf Masse, ist :

| | |
|---|---|
| Saccharose | 10 bis 20 % |
| Mono-Allyldisaccharid | 25 bis 35 % |
| Di-Allyldisaccharid | 20 bis 30 % |
| Tri-Allyldisaccharid | 10 bis 20 % |
| Tetra-Allyldisaccharid | 5 bis 7 % |
| Allyldisaccharid mit einem Substitutionsgrad über oder gleich 5 | < 3.5 % |

16. Verfahren zur Herstellung eines Copolymers nach Anspruch 15, dadurch gekennzeichnet, daß das wäßrige Allyldisaccharid-Gemisch, auf Masse bezogen, enthält :

30 bis 50 % Wasser
20 bis 30 % eines Alkalimetallhalogenids
20 bis 50 % Allyldisaccharid.

17. Verfahren zur Herstellung eines Copolymers nach einem der Ansprüche 12 bis 16, dadurch gekennzeichnet, daß das wäßrige Gemisch einen pH-Wert von etwas unter 7 hat.

18. Verfahren zur Herstellung eines Copolymers nach Anspruch 11, dadurch gekennzeichnet, daß die Trocknung durch Waschen mit Methanol und anschließendem Durchgang durch eine Trockenkammer gesichert wird.

19. Verfahren zur Herstellung eines Copolymers nach Anspruch 11, dadurch gekennzeichnet, daß das Copolymer anschließend geliert, ausgefällt und mit Methanol oder Ethanol gewaschen wird.

20. Verfahren zur Herstellung eines Copolymers nach Anspruch 11 oder 19, dadurch gekennzeichnet, daß das Copolymer außerdem gefriergetrocknet wird.

21. Verwendung der Copolymere nach einem der Ansprüche 1 bis 10 als Zusätze zu den absorbierenden Tampons von Damenbinden, Babywindeln, Windeln für an Inkontinenz leidenden Erwachsenen.